# DEMANDE DE BREVET EUROPEEN

(11) **EP 4 821 499 A1**
(43) Date de publication de la demande: **14.10.2026**
(21) Numéro de dépôt: 26170851.5
(22) Date de dépôt: 07.04.2026
(51) Int. Cl.: A61K 31/60, A61K 8/92, A61K 8/9789, A61K 9/00, A61K 9/107, A61K 36/22, A61K 47/02, A61K 47/12, A61K 47/44, A61P 17/02, A61P 17/12, A61P 29/00, A61Q 19/00

(54) **COMPOSITION PHARMACEUTIQUE COMPRENANT DE L ACIDE ANACARDIQUE POUR SON UTILISATION EN TANT QUE COAGULANT CELLULAIRE**

(30) Priorité: 07.04.2025 FR 2503646
(71) Demandeur: Ephyla, 56190 Arzal (FR)
(72) Inventeur: BOURGETEAU, Vincent, 56130 FEREL (FR)
(74) Mandataire: Eveillard, Sophie

(57) **Abrégé**

L'invention vise une composition pharmaceutique comprenant de l'acide anacardique pour son utilisation en tant que coagulant cellulaire, notamment pour exfolier la peau. Les utilisations visent notamment la coagulation des adipocytes pour conférer un effet anti-cellulite à la peau, et/ou permettre la réduction de lipomes, la coagulation cellulaire dans l'environnement cellulaire en inflammation autour des cellules nerveuses pour agir en tant qu'agent anti-douleur, la coagulation pour réduire une fibrose ou un kyste.

## Description

La présente invention se rapporte au domaine de la coagulation cellulaire encore appelée « dermo-coagulation » et plus particulièrement à une composition pharmaceutique comprenant de l'acide anacardique pour son utilisation en tant que coagulant cellulaire ou dermo coagulant, notamment pour l'exfoliation profonde de la peau.

La peau est la principale barrière de protection du corps humain à l'égard des agressions extérieures telles que la pollution atmosphérique, les variations climatiques, le rayonnement UV et également à l'égard des antigènes potentiellement nocifs. La peau est constituée de trois couches : l'épiderme, le derme et l'hypoderme.

L'épiderme, qui est la couche superficielle en contact avec l'extérieur, est plus particulièrement concerné par les interactions avec le milieu extérieur. L'épiderme est recouvert par un film hydrolipidique et est composé de quatre couches : la couche cornée, la couche granuleuse, la couche épineuse et la couche basale. L'épiderme est normalement constitué de quatre types de cellules : les kératinocytes qui représentent 80% de l'ensemble de ses cellules, les 20% restant étant composés des mélanocytes, des cellules de Langerhans ou cellules immunocompétentes et des cellules de Merkel, ces trois groupes de cellules étant dispersées entre les kératinocytes.

Le derme est la couche épaisse de la peau, c'est un tissu de type conjonctif contenant un réseau très abondant de vaisseaux sanguins. Le derme est composé :
de macromolécules de type protéique, en particulier de fibres de collagène, d'élastine et de fibronectine, qui vont constituer un socle pour l'épiderme ;
de mucopolysaccharides dans lequel baignent les macromolécules, les mucopolysaccharides comprennent des glycosaminoglycanes qui sont des protéines aptes à capter l'eau dans le derme et constituer un « réservoir » d'hydratation ;
des cellules notamment les fibroblastes, des vaisseaux sanguins constitués de cellules endothéliales et des cellules du système immunitaire.

Le derme joue un rôle primordial dans la cicatrisation et dans l'élimination de produits toxiques.

Enfin l'hypoderme, situé sous le derme, est la couche la plus profonde de la peau. Il correspond au tissu adipeux riche en adipocytes.

Par « coagulation cellulaire », on entend la prise en masse solide ou semi-solide d'un tissu, ou un liquide cellulaire, organique ; la coagulation cellulaire est un phénomène qui passe par l'apoptose ou mort cellulaire.

Sans vouloir être lié à une quelconque théorie, l'utilisation selon la présente invention opère par voie intracellulaire, et affecte principalement les cellules déviantes ou anormales ou encore des « cellules en mauvaises santé » ; dans certains cas, elle peut aussi faire coaguler des cellules saines.

Le problème à l'origine de la présente invention était de proposer un nouvel agent apte à permettre la coagulation cellulaire conduisant à une exfoliation de la peau, notamment au niveau du derme et/ou de l'épiderme. Dans de nombreux cas, la coagulation cellulaire va conduire à la formation d'une croûte au niveau de la peau.

Parmi les procédés physiques d'exfoliation du derme, on connait déjà les procédés de dermo-coagulation laser, thermo-dermo-coagulation, electro-dermo-coagulation ou cryo-dermo-coagulation. Ces méthodes sont efficaces cependant elles conduisent généralement à la destruction des cellules et sont souvent douloureuses et/ou stressantes pour l'utilisateur. En outre, comme déjà mentionné, l'utilisation selon la présente invention vise principalement les cellules déviantes ou anormales ou encore des « cellules en mauvaises santé ».

L'exfoliation du derme par voie chimique est essentiellement réalisée avec des compositions à base de phénols. Ces solutions présentent néanmoins des inconvénients en particulier en termes de sensibilisation de la peau.

Il demeure toujours un besoin d'une nouvelle solution d'exfoliation du derme profond voire de l'hypoderme qui soit plus douce, moins invasive et moins stressante et qui vise principalement les cellules moribondes ou défaillantes.

De manière inattendue et avantageuse, l'inventeur a montré que ce problème de la coagulation cellulaire pouvait être résolu au moyen d'acide anacardique.

### Résumé de l'invention

Un premier aspect de l'invention vise une composition pharmaceutique comprenant de l'acide anacardique pour son utilisation en tant que coagulant cellulaire. La composition pharmaceutique selon l'invention présente l'avantage d'être apte à atteindre le derme voire l'hypoderme, même par application topique superficielle.

Selon un premier aspect, l'invention vise ladite composition pharmaceutique pour son utilisation pour traiter les adipocytes et conférer un effet anti-cellulite à la peau, et/ou permettre la réduction de lipomes.

Selon un deuxième aspect, l'invention vise ladite composition pharmaceutique pour son utilisation pour traiter l'environnement cellulaire en inflammation autour des cellules nerveuses et agir en tant qu'agent anti-douleur.

Selon un troisième aspect, l'invention vise ladite composition pharmaceutique pour son utilisation pour réduire une fibrose ou un kyste.

Selon un quatrième aspect, l'invention vise ladite composition pharmaceutique pour son utilisation pour exfolier la peau, et/ou les défauts de surfaces notamment les lentigos, les acrochordons (fibromes mous ou fibromes pendulaires), les kystes cutanés, les molluscums, les kératoses et les xanthélasmas.

De préférence, l'invention vise ladite composition pharmaceutique pour son utilisation pour exfolier la peau dans au moins un traitement choisi dans le groupe formé par :
le traitement des cicatrices en creux, le curetage et le traitement des verrues, le traitement des boutons d'herpès, le traitement des acrochordons (fibromes mous ou fibromes pendulaires), le traitement des taches pigmentaires ou lentigos, le traitement des kératoses, le traitement des xanthélasmas, le curetage des plaies, le traitement des télangiectasies, le traitement des varicosités, le traitement des angiomes, le traitement des taches rubis, le traitement des vaisseaux sanguins superficiels de la peau.

Les compositions utilisées selon l'invention sont en outre stables, non irritantes, non toxiques, non allergisantes pour la peau.

D'autres aspects, avantages, propriétés de la présente invention sont présentés dans la description et les exemples qui suivent.

### Description détaillée

### Définitions

Dans le présent texte, sauf indications contraires spécifiques, les pourcentages sont exprimés en poids d'une composition de référence.

Dans le présent texte, les intervalles sont définis de façon abrégée afin d'éviter de décrire chacune des valeurs et toutes les valeurs de cet intervalle, cependant toute valeur appropriée dans l'intervalle peut être choisie comme la valeur supérieure, la valeur inférieure ou les valeurs terminales de l'intervalle. Par exemple, un intervalle de 0,1 à 1,0 représente les valeurs terminales de 0,1 et 1,0, ainsi que les valeurs intermédiaires de 0,2 ; 0,3 ; 0,4 ; 0,5 ; 0,6 ; 0,7 ; 0,8 ; 0,9 et tous les intervalles intermédiaires compris dans 0,1 à 1,0, tels que 0,2 à 0,5 ; 0,2 à 0,8 ; 0,7 à 1,0... Sauf indications contraires, un intervalle défini comme étant « compris entre la valeur A et la valeur B » inclut les valeurs A et B et est donc équivalent à un intervalle « allant de la valeur A à la valeur B » , l'expression « au moins » comprend la valeur énoncée après, par exemple, «au moins 5 % » doit être compris comme comprenant également « 5 % », l'expression « un maximum de » comprend la valeur énoncée après, par exemple, « un maximum de 5 % » doit être compris comme comprenant également « 5 % ».

En outre, dans le présent texte, les valeurs mesurables, telles qu'une quantité, doivent être comprises comme comprenant les écarts types qui peuvent être facilement déterminés par l'homme du métier dans le domaine technique de référence. De préférence, ces valeurs sont destinées à comprendre des variations de ± 5 %.

Par « peau » on entend l'épiderme, le derme et l'hypoderme du visage ou du corps ou du cuir chevelu.

### Acide anacardique

L'acide anacardique est un mélange de plusieurs molécules présentant la structure suivante : un noyau correspondant à l'acide salicylique comprenant un radical -CH₂-R, le radical R étant un radical hydrocarboné en C14 comprenant de 0 à 3 insaturations. En particulier l'acide anacardique comprend les quatre dérivés suivants d'acide 6-alkylsalicylique de formule I :

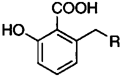

| nombre de C, nombre d'insaturations* | Nom |
|---|---|
| C15, 0 | Acide 6-n-pentadecylsalicylique |
| C15, 1 | Acide 6- [8(Z)-n-pentadecenyl] salicylique |
| C15, 2 | Acide 6- [8(Z), 11(Z)-n-pentadecadienyl] salicylique |
| C15, 3 | Acide 6- [8(Z), 11(Z), 14(Z)-n-pentadecatrienyl] salicylique |

| | |
|---|---|
| * :sur le radical CH2R | |

En particulier l'acide anacardique peut être un extrait de coques de noix de cajou.

Selon un mode particulier, l'acide anacardique utilisé dans la présente invention est choisi parmi l'acide 6-n-pentadecylsalicylique, l'acide 6-[8(Z)-n-pentadecenyl] salicylique, l'acide 6- [8(Z), 11(Z)-n-pentadecadienyl] salicylique, l'acide 6- [8(Z), 11(Z), 14(Z)-n-pentadecatrienyl] salicylique ainsi que leurs mélanges en toutes proportions, de manière particulière l'acide anacardique utilisé dans la présente invention est choisi parmi l'acide 6-n-pentadecylsalicylique, l'acide 6-[8(Z)-n-pentadecenyl] salicylique, ainsi que leurs mélanges en toutes proportions.

### Extrait de coques de noix de cajou

Selon un autre mode particulier, la composition selon la présente invention comprend un extrait de coques de noix de cajou ou *Anacardium occidentale* L., ledit extrait contenant de de l'acide anacardique.

Un extrait de coques de noix de cajou utilisable selon l'invention est susceptible d'être obtenu par un procédé de préparation comprenant au moins les étapes suivantes, dans cet ordre :
- fournir des coques de noix de cajou,
- broyer les coques de noix de cajou pour obtenir une taille moyenne des particules inférieure ou égale 5 mm,
- procéder à l'extraction des coques de noix de cajou au moyen d'éthanol à 96° à une température comprise entre 20°C et 45°C,
- filtrer,
- récupérer le filtrat,
- évaporer l'éthanol,
- récupérer l'extrait obtenu.

En particulier, cet extrait de coques de noix cajou comprend de 60 à 95 % en poids d'acide anarcardique par rapport au poids total de l'extrait. Il s'agit donc d'un extrait concentré en acide anarcardique.

Cet extrait de coques de noix cajou comprend de 50 à 90 % en poids d'acide 6- [8(Z)-n-pentadecenyl] salicylique par rapport au poids total de l'extrait.

L'extrait concentré d'acide 6- [8(Z)-n-pentadecenyl] salicylique, peut ensuite être distillé sous vide afin d'obtenir de l'acide 6- [8(Z)-n-pentadecenyl] salicylique à 98%.

### Composition

La composition pharmaceutique utilisée selon la présente invention peut comprendre de 0,1% à 100,0 % d'acide anacardique, par rapport au poids total de la composition.

Selon une première variante, la composition utilisée selon l'invention est une composition essentiellement huileuse.

Selon cette première variante, ladite composition utilisée contient de 0,1 à 99,9% en poids, par rapport au poids total de la composition, d'une phase huileuse comprenant au moins une huile choisie parmi les huiles végétales, le squalane, le squalène, les esters de corps gras végétaux ou obtenus par biotechnologie.

Selon un mode de réalisation préféré, la phase huileuse comprend au moins une huile végétale choisie dans le groupe formé par l'huile de tournesol, l'huile de colza, l'huile d'olive, l'huile de cameline, l'huile d'arachide, l'huile de coco, l'huile de pépin de raisin, l'huile de ricin, l'huile d'argan, l'huile de Djansang, l'huile de dattier du désert, l'huile de chanvre, l'huile de nigelle, l'huile de figue de barbarie, l'huile de macadamia, l'huile de soja, l'huile de palme et de palmier, l'huile de Tamanu, l'huile de Kukui (ou bancoulier), l'huile de sésame, l'huile de lin, l'huile de noix, l'huile de noisette, l'huile d'amande, l'huile de baobab, l'huile de fruit de la passion, l'huile de noix du Brésil, l'huile d'hibiscus, l'huile de pépin de courge, l'huile de Luffa, l'huile de Carapa, l'huile d'Onagre, l'huile de bourrache, huile d'avocat, l'huile d'argousier, l'huile de noyau d'abricot, l'huile de noyau de cerise, l'huile de pépin de pomme, l'huile de grenade, l'huile de Jojoba, l'huile de rose musquée, l'huile de prune, le beurre de karité, le beurre de cacao, le beurre de Kokum, le beurre de mangue, le beurre de moabi, le beurre de Karanja, le beurre de Tucuma, le beurre de Cupuaçu, le beurre de Buriti, le beurre de Murumuru, le beurre de Kombo, le beurre de Kpangnan et les triglycérides notamment d'acides caprylique/caprique.

Selon une deuxième variante particulière, la composition utilisée selon l'invention comprend une émulsion de Pickering H/E contenant une phase aqueuse, une phase huileuse végétale et un phyllosilicate organomodifié.

Les émulsions de Pickering sont des émulsions stabilisées par des particules solides. Lors de la préparation de l'émulsion, lesdites particules solides se positionnent à l'interface entre la phase aqueuse et la phase huileuse.

Les émulsions de Pickering utilisées selon la présente invention sont des émulsions huile dans eau, c'est-à-dire H/E, stabilisées par une argile particulière qui est un phyllosilicate naturel modifié.

Avantageusement, la phase aqueuse de l'émulsion de Pickering est présente en une quantité allant de 43% à 93%, de préférence 55% à 70% et de manière préférée 58% à 65% en poids par rapport au poids total de la composition.

Selon un mode de réalisation préféré, le phyllosilicate naturel modifié comprend un phyllosilicate choisi dans le groupe constitué par les vermiculites et les smectites. De préférence, le phyllosilicate peut être choisi dans le groupe constitué par les montmorillonites, bentonites, nonytronites, beidellites, volkonskoites, hectorites, saponites, sauconites, sobockites, stevensites, svinfordites, de préférence ce sont des phyllosilicates de sodium, de potassium, de calcium, ou de leurs mélanges. De manière plus préférée, le phyllosilicate est choisi dans le groupe constitué par l'hectorite, la montmorillonite, la bentonite ou leurs mélanges.

Selon un mode de réalisation préféré, le phyllosilicate naturel modifié comprend un composé organique choisi dans le groupe constitué par la gomme xanthane, la gomme guar, la gomme de Tara ou de Caroube, la gomme d'acacia, le carraghénane, l'alginate, le chitosane, la pectine, l'acide citrique, l'acide tartrique, l'acide oxalique, l'acide succinique, l'acide malique, l'acide acétique, l'acide lactique, l'acide propionique, l'acide salicylique, les glycosaminoglycanes. Avantageusement, le phyllosilicate naturel modifié comprend une bentonite modifiée par la gomme de xanthane et l'acide citrique.

Selon un autre mode de réalisation, le phyllosilicate naturel modifié comprend un composé organique choisi parmi tout composé organique connu dans l'art des émulsions de Pickering, tels que ceux divulgués dans le brevet français n° FR 2 976 503.

Avantageusement, le phyllosilicate modifié est présent en une quantité allant de 2% à 20%, de préférence 2,5% à 10% et de manière préférée 3,5% à 8% en poids par rapport au poids total de la composition.

Avantageusement, la phase huileuse de l'émulsion de Pickering est présente en une quantité allant de 5% à 45%, de préférence 10% à 35% et de manière préférée 15% à 30% en poids par rapport au poids total de la composition.

Le procédé de préparation de l'émulsion de Pickering comprend au moins les étapes suivantes dans cet ordre :
- préparer la phase aqueuse ;
- ajouter le phyllosilicate organomodifié à la phase aqueuse et mélanger ;
- ajouter la phase huileuse végétale au mélange obtenu et obtenir une émulsion de Pickering H/E.

Avantageusement la composition utilisée selon la présente invention comprend de 0,001 à 20% en poids d'au moins un agent choisi parmi les agents humectants, les épaississants, les agents de texture, les émulsifiants, les agents dispersants, les agents moussants, les émollients, les conservateurs, les colorants, les extraits de plantes, les fibres végétales, les minéraux, les agents correcteurs de pH, les principes actifs et les parfums.

Bien entendu, l'homme du métier veillera à choisir ces agents de manière à ne pas altérer les propriétés de la composition selon l'invention.

### Utilisations

L'invention vise une composition pharmaceutique comprenant de l'acide anacardique pour son utilisation en tant que coagulant cellulaire.

Avantageusement ledit coagulant cellulaire conduit à une nécrose et une abrasion de l'épiderme et du derme.

Selon un premier aspect, l'invention vise la composition pharmaceutique comprenant de l'acide anacardique pour son utilisation pour traiter les adipocytes et conférer un effet anti-cellulite à la peau, et/ou permettre la réduction de lipomes.

En effet, l'utilisation de l'acide anacardique permet la réduction et libération de graisses par coagulation des adipocytes et ainsi de conférer un effet anti-cellulite, et permettre la réduction de lipomes.

Selon un deuxième aspect, l'invention vise la composition pharmaceutique comprenant de l'acide anacardique pour son utilisation pour traiter l'environnement cellulaire en inflammation autour des cellules nerveuses et agir en tant qu'agent anti-douleur.

Selon un troisième aspect, l'invention vise la composition pharmaceutique comprenant de l'acide anacardique pour son utilisation pour réduire une fibrose ou un kyste.

Selon un deuxième mode l'invention vise la composition pharmaceutique comprenant de l'acide anacardique pour son utilisation pour exfolier la peau.

Comme montré dans les exemples ci-après, cette utilisation permet une exfoliation de la peau en profondeur, notamment au niveau du derme conduisant à une nécrose et une abrasion de l'épiderme et du derme.

Cette utilisation permet également la coagulation des cellules épidermiques c'est-à-dire formation d'une croûte.

De manière particulière, la composition pharmaceutique comprenant de l'acide anacardique est utilisée pour exfolier la peau dans au moins un traitement choisi dans le groupe formé par : le traitement des cicatrices en creux, le curetage et le traitement des verrues, le traitement des boutons d'herpès, le traitement des acrochordons (fibromes mous ou fibromes pendulaires), le traitement des taches pigmentaires ou lentigos, le traitement des kératoses, le traitement des xanthélasmas, le curetage des plaies, le traitement des télangiectasies, le traitement des varicosités, le traitement des angiomes, le traitement des taches rubis, le traitement des vaisseaux sanguins superficiels de la peau notamment au niveau du visage et du décolleté. Les exemples qui suivent visent à illustrer l'invention sans en limiter la portée.

### Exemples

### Exemple 1- Extraits de coques de noix de cajou

### 1.1 Préparation de l'extrait

Les coques de noix de cajou ont été découpées mécaniquement au moyen d'un broyeur à couteaux de manière à obtenir des particules de coques inférieures ou égales à 5 mm.

100 g de coques de noix de cajou découpées ont ensuite été introduites dans un réacteur avec 300 g d'éthanol à 96°.

Le cisaillement a ensuite été maintenu sous agitateur Misceo 4 couteaux pendant 2 heures à une température d'environ 40°C. La température du mélange ne doit pas dépasser 45°C notamment afin d'éviter l'altération de l'acide anacardique en cardanol.

A l'issue de ces 2 heures, le mélange a été filtré sur filtre plissé.

243,55 g de filtrat ont été obtenus, soit un rendement de 60,89%. L'extrait obtenu (filtrat) comprend un taux de matières sèches de 11,17% soit 27,20 g.

Puis le filtrat a été placé dans un rotavapor à 50°C où l'évaporation de l'éthanol a été effectuée sous vide. Après évaporation de l'éthanol (206,0g), 4,6 g d'extrait de coques de noix de cajou concentré ont été obtenus.

### Détermination de la composition de l'extrait concentré

Par analyse en chromatographie en phase gazeuse, il a pu être montré que l'extrait obtenu comprend 73% d'acide 6- [8(Z)-n-pentadecenyl] salicylique, correspondant à la formule I avec CH2R égal C15 :1. Cet extrait à 73% est nommé « extrait concentré » dans la suite du texte.

### 1.2 Préparation de l'extrait pur

L'extrait concentré à 73% d'acide 6- [8(Z)-n-pentadecenyl] salicylique, obtenu ci-dessus a été distillé par distillation moléculaire sous vide afin d'obtenir de l'acide 6- [8(Z)-n-pentadecenyl] salicylique à 98%. Cet extrait à 98% est nommé « extrait pur » dans la suite du texte.

### Exemple 2-Compositions pharmaceutiques

### Exemple 2.1 a Composition topique

Dans le tableau 1 est présentée une formulation topique lipophile utilisable selon l'invention

**[Tableau 1]**

| Phase | Nom commercial | % | INCI |
|---|---|---|---|
| A | Huile de tournesol raffinée commercialisée par CAUVIN | 28,00 | Helianthus annuus seed oil |
| A | Myritol 318 commercialisé par BASF | 28,00 | Caprylic /capric triglyceride |
| A | OLO3D commercialisé par Ephyla | 14,00 | Stearalkonium Bentonite |
| B | Extrait pur (98%) de coques de cajou obtenu à l'exemple 1.2 | 30,00 | *Anarcardium occidentale L* (Cashew shell) extract |
| Total | | 100 | |

Les constituants de la phase A ont été mélangés à 20°C au moyen d'un mixeur mécanique à couteaux ou un rotor stator à une vitesse comprise entre 3500 et 4000 rpm jusqu'à homogénéisation, c'est-à-dire environ 7 minutes.

Puis la phase A a été mélangée avec la phase B à 20°C au moyen d'un mixeur mécanique à couteaux ou un rotor stator à une vitesse comprise entre 3500 et 4000 rpm jusqu'à homogénéisation (environ 5-7 minutes).

Cette formule est strictement lipophile, elle permet à l'acide anacardique de pénétrer en profondeur pour atteindre le derme puis l'hypoderme, cette formule permet ainsi de coaguler les cellules des couches profondes de la peau ; cette formule a la même fonction qu'un patch topique, elle s'applique en couche sensiblement épaisse pour mimer un patch.

Cette composition permet de traiter les couches profondes du derme et de l'hypoderme, elle permet de faire coaguler les cellules pour une mise en nécrose.

Cette composition a été appliquée sur des patients (volontaires) présentant des verrues profondes ou des kystes graisseux dans l'hypoderme ou de la cellulite.

Ainsi 3 patients ont été traités avec cette composition **(exemple 2.1 a) :** 1 patient avec une verrue plantaire d'un diamètre de 7-8 mm ; 1 patient avec un kyste graisseux au niveau de l'hypoderme du bras d'un diamètre de 6-7 mm ; 1 patient avec de la cellulite sur le haut des bras.

### Exemple 2.1 b-traitements

### Matériel et méthode :

Les observations ont été effectuées avec un appareil dermatoscope de type FIREFLY Digital DE300 (zoom x60).

### Préparation des patients

La préparation des 3 patients à traiter a été effectuée comme suit :
1. Remplir la fiche de suivi : sexe, âge, type de tache à traiter.
2. Mettre des gants de laboratoire.
3. Nettoyer la zone à traiter avec de l'éthanol 70 %.
4. Observer initialement la zone avec le dermatoscope de type FIREFLY Digital DE300 (zoom x60).

### Application du traitement

L'application du traitement a été effectuée comme suit :
1. Centrer la tache dans le trou du pansement "protecteur cors" et le coller sur la peau.
2. Ouvrir le mini-flacon seringue à l'opercule de l'aiguille comprenant la composition à appliquer.
3. Déposer la composition dans le trou du pansement, au contact direct de la peau.
4. Refermer aussitôt l'opercule et conserver le flacon au réfrigérateur.
5. Recouvrir le trou du pansement avec un sparadrap, puis fixer l'ensemble en formant une croix avec deux autres bandes de sparadrap, dépassant largement le premier pansement pour une bonne adhérence.
6. Temps de pose : 6 heures.
7. Mettre des gants pour retirer les sparadraps et le pansement.
8. Éviter tout étalement de la composition sur la peau environnante.
9. Rincer la zone traitée avec une pipette de sérum physiologique ou à l'eau claire froide.
10. Sécher délicatement à l'aide d'une compresse.

### Soin du traitement

1. Recouvrir la zone d'un pansement ouaté propre.
2. Changer le pansement tous les jours pendant 10 à 14 jours selon l'évolution locale

### Suivi et documentation

1. Observation quotidienne avec le dermatoscope de type FIREFLY Digital DE300 (zoom x60) pendant les 4 premiers jours.
2. Puis observation tous les 2-3 jours jusqu'au jour J+14.

### Observations sur le patient avec verrue plantaire

1. J0 : après 6h ; la verrue plantaire s'est teintée et a pris du relief ;
2. J1 : Cornéification du tissu contaminé par la verrue plantaire ;
3. J2 : inflammation profonde et du pourtour de la zone traitée ; cornéification confirmée de la verrue plantaire ;
4. J3 : inflammation profonde marquée ainsi que du pourtour de la zone traitée ; cornéification confirmée de la verrue plantaire que se surélève : l'inflammation profonde semble pousser vers le haut la verrue ;
5. J5 : inflammation profonde marquée ainsi que du pourtour de la zone traitée ; durcissement/ densification de la verrue plantaire ; curetage au scalpel de la verrue durcie en surface ; élimination de près de 75% de la taille de la verrue ;
6. J7 : réduction de l'inflammation profonde et du pourtour de la zone traitée (régression de l'état inflammatoire), densification du tissu porteur de la verrue très réduite, et curetage au scalpel de ce reste de verrue ; réduction totale de la verrue plantaire ;
7. J10 : absence d'inflammation, la peau apparaissait saine et légèrement en creux (moins d'hypoderme et moins de derme en épaisseur) ; la verrue n'était plus visible.

### Observations sur le patient avec kyste graisseux hypodermique

1. J0 : après 6h ; la zone traitée était rougie par une activation de la circulation sanguine (hyperémie modérée à significative) ; le kyste graisseux était toujours perceptible ;
2. J1 : hyperémie marquée avec léger gonflement de la peau (inflammation profonde) ; kyste graisseux toujours perceptible ;
3. J2 : hyperémie marquée, gonflement de la peau (inflammation profonde) ; le kyste graisseux était toujours perceptible mais moins dense ;
4. J3 : idem à J2 avec léger effet de diffusion autour de la zone traitée ; le kyste graisseux était toujours perceptible mais moins dense ;
5. J5 : idem J3 avec hyperémie marquée sur le pourtour de la zone traitée ; kyste graisseux était toujours perceptible mais réelle perte de densité dense ;
6. J7 : réduction de l'inflammation profonde et du pourtour de la zone traitée (régression de l'état inflammatoire), au toucher le kyste graisseux n'était plus rond, bombé mais mou en s'aplatissant au toucher ;
7. J10 : inflammation légère sur zone traitée et son pourtour, le kyste graisseux était très peu perceptible, il disparaissait ;
8. J14 : absence d'inflammation, la peau semblait saine et était légèrement en creux sur la zone traitée (moins d'hypoderme et moins de derme en épaisseur) ; le kyste avait disparu.

### Observations sur le patient avec cellulite

1. J0 : après 6h ; la zone traitée était rougie par une activation de la circulation sanguine (hyperémie modérée à significative) ;
2. J1 : hyperémie marquée avec léger gonflement de la peau (inflammation profonde) ;
3. J2 : hyperémie marquée : gonflement de la peau (inflammation profonde) ;
4. J3 : idem à J2 avec léger effet de diffusion autour de la zone traitée ;
5. J5 : idem J3 avec hyperémie marquée sur le pourtour de la zone traitée ;
6. J7 : réduction de l'inflammation profonde et du pourtour de la zone traitée (régression de l'état inflammatoire), la cellulite n'était plus perceptible ;
7. J10 : inflammation légère sur zone traitée et son pourtour, la cellulite n'était plus perceptible au contraire sensation de creux : l'hypoderme semble s'être affiné ;
8. J14 : absence d'inflammation, la peau semblait saine et légèrement en creux sur la zone traitée : beaucoup moins d'hypoderme et moins de derme en épaisseur ; la cellulite avait disparu.

Cette composition est aussi utilisable dans les traitements visant à éliminer la graisse hypodermique, notamment les kystes graisseux par nécrose et/ou les traitements visant à éliminer les verrues profondes, de type verrue plantaire, par nécrose.

### Exemple 2.2 a composition topique

Dans le tableau 2 est présentée une composition utilisable selon l'invention contenant une émulsion de pickering dans laquelle la phase huileuse est gélifiée

**[Tableau 2]**

| Phase | Nom commercial | % | INCI |
|---|---|---|---|
| A | Eau | Qsp | Aqua |
| A | Frametime CX commercialisé par Ephyla | 4,50 | Bentonite & xanthan gum & citric acid |
| A | Novasolve DMI | 3,00 | Dimethyl isosorbide |
| A | Propanediol | 6,00 | Propanediol |
| A | Sorbitol | 0,80 | Sorbitol |
| B | OLO3D commercialisé par Ephyla | 4,00 | Stearalkonium Bentonite |
| B | Extrait pur (98%) de coques de cajou obtenu à l'exemple 1.2 | 30,00 | *Anarcardium occidentale L* (Cashew shell) extract |
| Total | | 100 | |

D'une part, les constituants de la phase A ont été mélangés à 20°C avec un mixeur mécanique à couteaux ou un rotor stator à une vitesse comprise entre 3500 et 4000 rpm durant 7 minutes pour obtenir un mélange homogénéisé. D'autre part, les constituants de la phase B ont été mélangés à 20°C avec un mixeur mécanique à couteaux ou un rotor stator à une vitesse comprise entre 3500 et 4000 rpm durant 7 minutes

Enfin la phase B a été introduite dans la phase A et les phases ont été mélangées avec un mixeur mécanique à couteaux ou un rotor stator à une vitesse de 4000 rpm durant 5-7 minutes pour obtenir une composition homogénéisée.

Le pH de la composition obtenue est ajusté à 6,5-7,5.

Cette composition comprend une émulsion de pickering où la phase huileuse de la phase B est gélifiée avec le « Stearalkonium Bentonite », puis encapsulée dans des capsules d'une dizaine de microns de diamètre. Cette gélification de l'extrait pur de coque de cajou suivie d'une encapsulation de type capsule d'huile dans eau, permet à l'acide anacardique de se libérer très progressivement à la surface de la peau et de traiter principalement les couches superficielles de la peau à savoir l'épiderme.

Le mode d'application privilégié est d'utiliser un pansement percé qui délimite la zone à traiter, « l'œillet » ou le « trou » du pansement indique la zone à traiter, puis d'introduire la composition dans le trou ou l'œillet au contact de la peau et recouvrir le trou ou l'œillet avec un sparadrap ; laisser le dispositif en place durant au minimum 30 minutes et jusque 24 h, puis éliminer le dispositif appliqué et éventuellement, rincer la peau à l'eau claire de l'excédent de produit.

3 patients ont été traités avec cette composition (exemple 2.2a) ; 1 patient avec un acrochordon au niveau du cou d'une longueur de 4 mm avec un diamètre de 1 mm, la zone pigmentaire à sa base faisait un diamètre d'environ 2-3 mm ; 1 patient avec un Angiome rubis vascularisé, de 1-2 mm d'épaisseur sur un diamètre de 5-6 mm sur le bras ; 1 patient avec un Naevus dysplasique irrégulier avec zone très foncée en relief de diamètre 4-7 mm.

### Exemple 2.2 b-traitements

### Matériel et méthode :

Les observations ont été effectuées avec un appareil dermatoscope de type FIREFLY Digital DE300 (zoom x60).

### Préparation des patients

La préparation des 3 patients à traiter a été effectuée de la même façon qu'à l'Exemple 2.1 b-traitements.

### Application du traitement

L'application du traitement a été effectuée de la même façon qu'à l'Exemple 2.1 b-traitements.

### Soin du traitement

Le soin du traitement a été effectuée de la même façon qu'à l'Exemple 2.1 b-traitements.

### Suivi et documentation

1. Observation quotidienne avec le dermatoscope de type FIREFLY Digital DE300 (zoom x60) pendant les 4 premiers jours.
2. Puis observation tous les 2-3 jours jusqu'au jour J+14.

### Observations sur le patient avec acrochordon (fibrome mou ou fibrome pendulaire)

1. J0 : après 5h ; la zone traitée était brunie par un effet de cornéification ; l'acrochordon s'était rigidifié, il apparaissait sensiblement rabougri ;
2. J1 : l'acrochordon était rigidifié ; la zone traitée s'était cornéifiée ;
3. J2 : L'acrochordon était décroché ; la zone traitée s'était durcie, le pourtour semblait plus vascularisé ;
4. J3 : idem à J2 avec léger effet hyperémiant de diffusion autour de la zone traitée ;
5. J5 : la zone traitée avait évolué en croûte ; le pourtour était toujours un peu rouge ;
6. J7 : la zone traitée était devenue une croûte qui séchait ;
7. J10 : la croûte était sèche et semblait se décoller sur son pourtour ;
8. J14 : la croûte s'était décollée ; la peau était saine sans cicatrice apparente ; la pigmentation et l'acrochordon (fibromes mou ou fibrome pendulaire) avaient disparu.

### Observations sur le patient avec Angiome rubis vascularisé,

1. J0 : après 5h ; la zone traitée était brunie par un effet de cornéification ; la vascularisation semblait enflammée ;
2. J1 : la zone traitée s'était cornéifiée ; la vascularisation avait coagulé et s'était rigidifiée ;
3. J2 : idem à J1 ;
4. J3 : idem à J2 avec léger effet hyperémiant de diffusion autour de la zone traitée ;
5. J5 : la zone traitée avait évolué en croûte ; le pourtour était toujours un peu rouge ;
6. J7 : la zone traitée était devenue une croûte qui séchait ;
7. J10 : la croûte était sèche et semblait se décoller sur son pourtour ;
8. J14 : la croûte s'était décollée ; la peau était saine sans cicatrice apparente ; la pigmentation et l'Angiome rubis avaient disparu.

### Observations sur le patient avec Naevus dysplasique

1. J0 : après 6-7h ; la zone traitée était brunie par un effet de cornéification ; la vascularisation semblait enflammée sur la zone en relief du Naevus ;
2. J1 : la zone traitée s'était cornéifiée ; la vascularisation avait coagulé et s'était rigidifiée ;
3. J2 : idem à J1 ;
4. J3 : idem à J2 avec léger effet hyperémiant de diffusion autour de la zone traitée ;
5. J5 : la zone traitée avait évolué en croûte ; le pourtour était en hyperémie rouge ;
6. J7 : la zone traitée était devenue une croûte qui séchait ; le pourtour était en hyperémie rouge comme en J5 ;
7. J10 : la croûte était sèche et semblait se décoller sur son pourtour ; le pourtour présentait toujours une hyperémie ;
8. J14 : la croûte s'était décollée ; la peau était saine sans cicatrice apparente ; le Naevus dysplasique avait disparu ; une hyperémie sur le pourtour était toujours présente mais de faible intensité.

Il s'agit donc d'une coagulation entrainant une exfoliation ; le traitement est particulièrement efficace pour éliminer par nécrose et exfoliation les acrochordons (fibromes mous ou fibromes pendulaires), les cicatrices, les vergetures, les lésions pigmentaires (mélasma), les Naevus notamment dysplasiques, les taches de vieillesse ou lentigos, les grains de beauté disgracieux tels que les angiomes rubis ou les Naevus dysplasiques. La coagulation des micro-vaisseaux sanguins observée dans le cas des angiomes rubis permet aussi des applications visant à stopper les saignements notamment lors de coupures cutanées touchant le derme et/ou l'hypoderme.

Il est à préciser que le contenu de cette composition pénètre à travers l'épiderme pour rejoindre le derme également, ce qui permet, chez d'autres volontaires, au niveau de la jonction dermo-épidermique, d'inhiber la douleur par nécrose cellulaire des cellules sous inflammation au niveau des terminaisons nerveuses, cette composition est donc particulièrement indiquée pour le curetage des plaies, en ce cas l'application n'excède pas 4 h et au minimum 1h : l'application en cataplasme agit, sans action mécanique, comme un curetage.

### Exemple 2.3 a composition topique

Dans le tableau 3 est présentée une composition utilisable selon l'invention contenant une émulsion de pickering dans laquelle la phase huileuse est gélifiée.

**[Tableau 3]**

| Phase | Nom commercial | % | INCI |
|---|---|---|---|
| A | Eau | Qsp | Aqua |
| A | Frametime CXG commercialisé par Ephyla | 5,0 | Bentonite & xanthan gum & Sodium stearoyl glutamate & citric acid |
| A | Benzoate de Sodium | 0,40 | Sodium benzoate |
| A | Acide citrique monohydraté | 0,40 | Citric acid |
| B | OLO3D commercialisé par Ephyla | 4,50 | Stearalkonium Bentonite |
| B | Extrait concentré (73%) de coques de cajou obtenu à l'exemple 1.1 | 35,00 | *Anarcardium occidentale L* (Cashew shell) extract |
| Total | | 100 | |

D'une part, les constituants de la phase A ont été mélangés à 20°C avec un mixeur mécanique à couteaux ou un rotor stator à une vitesse comprise entre 3500 et 4000 rpm durant 7 minutes pour obtenir un mélange homogénéisé. D'autre part, les constituants de la phase B ont été mélangés à 20°C avec un mixeur mécanique à couteaux ou un rotor stator à une vitesse comprise entre 3500 et 4000 rpm durant 7 minutes

Enfin la phase B a été introduite dans la phase A et les phases ont été mélangées avec un mixeur mécanique à couteaux ou un rotor stator à une vitesse de 4000 rpm durant 5-7 minutes pour obtenir une composition homogénéisée.

### Le pH de la composition obtenue est ajusté à 5,0-5,5.

Cette composition comprend une émulsion de pickering où la phase huileuse (phase B) est gélifiée avec le « Stearalkonium Bentonite », puis encapsulée dans des capsules d'environ 5 à 10 microns de diamètre. Cette gélification de l'extrait concentré de coques de cajou suivie d'une encapsulation de type capsules dans eau, permet à l'acide anacardique de se libérer progressivement à la surface de la peau et de traiter principalement les couches superficielles de la peau à savoir l'épiderme et de coaguler les cellules.

Le mode d'application privilégié est d'utiliser un pansement percé qui délimite la zone à traiter, « l'œillet » ou le « trou » du pansement indique la zone à traiter, d'introduire la composition dans le trou ou l'œillet au contact de la peau et recouvrir le trou ou l'œillet avec un sparadrap, laisser le dispositif en place durant au minimum 30 minutes et jusque 24 h, puis éliminer le dispositif appliqué et éventuellement, rincer la peau à l'eau claire de l'excédent de produit.

Après application sur des patients volontaires, on a observé que l'épiderme coagule et se transforme en croûte après 48 à 96 heures, la croûte disparaît ensuite au bout de 10 à 14 jours environ en laissant une peau cicatrisée nette et sans défaut : le problème ou le défaut a disparu dans la croûte. Il s'agit donc d'une exfoliation ; le traitement est particulièrement efficace pour éliminer par nécrose et exfoliation les acrochordons (fibromes mous ou fibromes pendulaires), les cicatrices, les vergetures, les lésions pigmentaires (mélasma), les Naevus notamment dysplasiques, les taches de vieillesse ou lentigos, les grains de beauté disgracieux tels que les angiomes rubis. La coagulation des micro-vaisseaux sanguins observée dans le cas des angiomes rubis permet aussi des applications visant à stopper les saignements notamment lors de coupures cutanées touchant le derme et/ou l'hypoderme.

Il a aussi été observé que cette composition pénètre en surface rapidement ce qui permet, chez d'autres volontaires, d'inhiber la douleur par nécrose cellulaire au niveau de la peau, cette composition est particulièrement indiquée aussi pour le curetage des plaies, dans ce cas l'application n'excède pas 4 h et au minimum 1h : l'application en cataplasme agit, sans action mécanique, comme un curetage.

Ainsi 4 patients ont été traités avec cette composition (exemple 2.3 a) ; 1 patient avec un Naevus dysplasique irrégulier avec une zone très foncée en relief de diamètre 4-7 mm sur le haut du dos ; 1 patient avec une verrue papillome sur le bras de forme ovale d'environ 7 mm de long et de 3-4 mm de large ; 1 patient avec un acrochordon (fibrome mou ou fibrome pendulaire) dans le pli du bras sur Naevi de longueur 5 mm, la tâche (Naevi) faisant un diamètre de 4 mm en surface de la peau ; 1 patient avec un Angiome rubis (vascularisé).

### Exemple 2.3 b-traitements

### Matériel et méthode :

Les observations ont été effectuées avec un appareil dermatoscope de type FIREFLY Digital DE300 (zoom x60).

### Préparation des patients

La préparation des 3 patients à traiter a été effectuée de la même façon qu'à l'Exemple 2.1 b-traitements.

### Application du traitement

L'application du traitement a été effectuée de la même façon qu'à l'Exemple 2.1 b-traitements.

### Soin du traitement

Le soin du traitement a été effectuée de la même façon qu'à l'Exemple 2.1 b-traitements.

### Suivi et documentation

1. Observation quotidienne avec le dermatoscope de type FIREFLY Digital DE300 (zoom x60) pendant les 4 premiers jours.
2. Puis observation tous les 2-3 jours jusqu'au jour J+14.

### Observations sur le patient avec Naevus dysplasique :

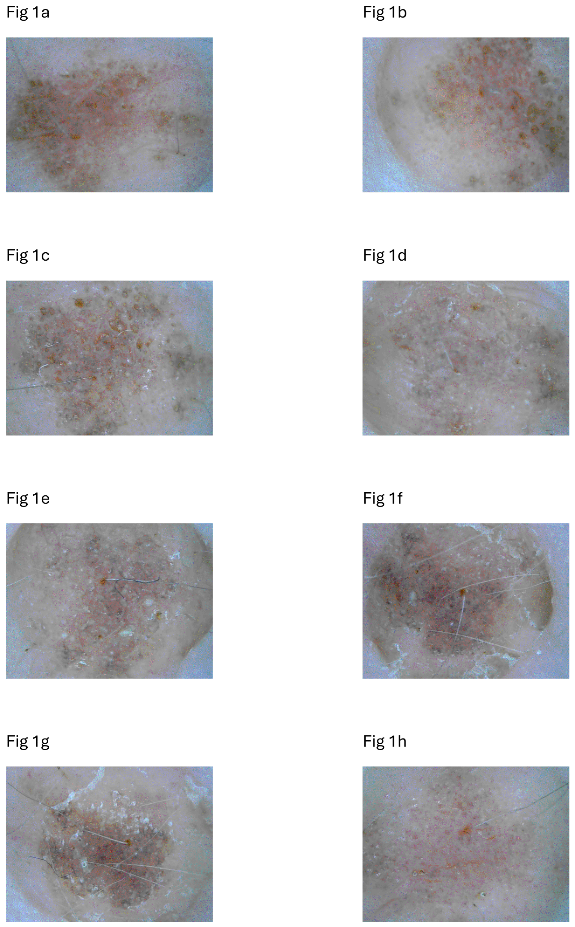

Les photographies des figures 1a-1h correspondent à ces observations :
1. J0 : avant application : Naevus dysplasique de 4 mm de long sur 4 mm de large, bombé et vascularisé sur la zone gauche (avec hyperpigmentation marquée) et centrale - figure 1a ;
2. J0 après rinçage/séchage : pas de sensation/douleur particulière ; agglutination de la pigmentation en surface, légère inflammation de la vascularisation - figure 1b ;
3. J1 ; Pas de sensation/douleur particulière ; agglutination de la pigmentation en surface et cornéification de l'épiderme, inflammation de la vascularisation - figure 1c ;
4. J2 : Pas de sensation/douleur particulière ; cornéification et exfoliation de l'épiderme ; forte réduction de l'inflammation vasculaire et probable coagulation vasculaire - figure 1d ;
5. J3 : Démangeaison sur le pourtour de la zone traitée ; exfoliation de l'épiderme ; disparition de la vascularisation (coagulation probable) - figure 1e ;
9. J7 : Démangeaison très modérée ; formation d'une croûte fine en surface de la zone traitée avec exfoliation sur le pourtour de la zone - figure 1 f ;
10.J10 : Très légère démangeaison probablement liée à la présence d'une fine croûte de peau sur la zone traitée ; la croûte de surface sèche, le pourtour termine de s'exfolier - figure 1g ;
11.J15 : Pas de sensation/douleur particulière ; le Naevus dysplasique a disparu ; trace de cicatrisation en profondeur au niveau du point de vascularisation central - figure 1h.

### Observations sur le patient avec une verrue :

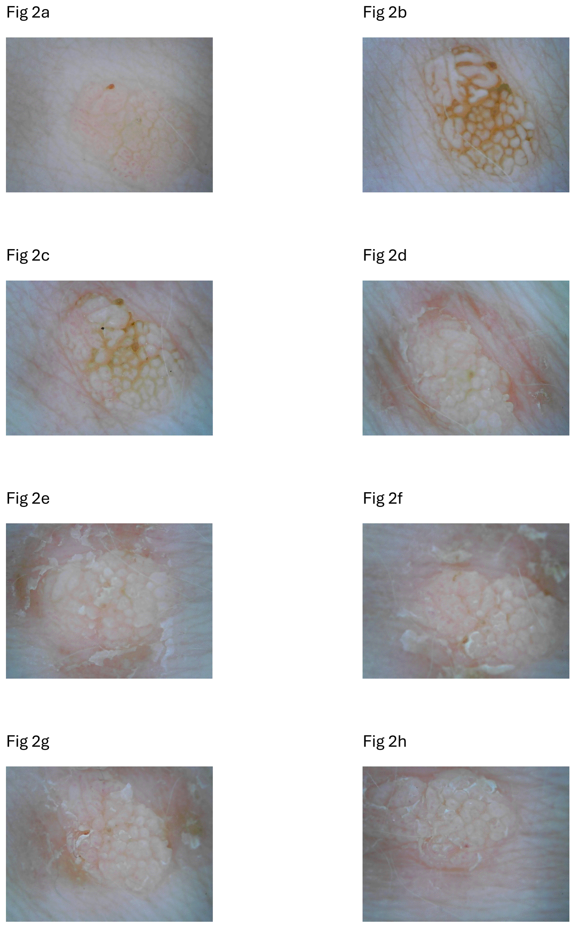

Les photographies des figures 2a-2h correspondent à ces observations :
1. J0 : avant application : verrue plate de surface - figure 2a ;
2. J0 après rinçage/séchage : restes de la formulation visibles (incrustation de la galénique dans la partie invaginée de la verrue) ; pas de sensation/douleur particulière - figure 2b ;
3. J1 : Restes de la formulation encore visibles (incrustation de la galénique dans la partie invaginée de la verrue) ; pas de sensation/douleur particulière bien que présence d'un halo de rougeur autour de la zone traitée ; nécrose/ exfoliation de la surface de la verrue et de l'épiderme- figure 2c ;
4. J2 : Légère démangeaison notamment au niveau du halo de rougeur autour de la zone traitée ; nécrose et exfoliation de l'épiderme au niveau de cette zone (halo) ; exfoliation de la surface de la zone traitée - figure 2d ;
5. J3 : Pas de sensation/douleur particulière ; nécrose & exfoliation de l'épiderme au niveau de cette zone (halo) ; exfoliation de la surface de la zone traitée - figure 2 e ;
6. J7 : Pas de sensation/douleur particulière ; nécrose & exfoliation de l'épiderme au niveau de cette zone (halo) ; poursuite de la nécrose & exfoliation de la surface de la zone traitée - figure 2 f ;
7. J10 : Pas de sensation/douleur particulière ; exfoliation de l'épiderme au niveau de cette zone (halo) ; poursuite de la nécrose & exfoliation de la surface de la zone traitée - figure 2g ;
8. J15 : Pas de sensation/douleur particulière ; exfoliation de l'épiderme au niveau de cette zone (halo) ; poursuite de l'exfoliation de la surface de la zone traitée ; la verrue continue à réagir et régresser alors que la peau sur le pourtour semble avoir retrouvé son état normal - figure 2h.

### Observations sur le patient avec un acrochordon (Fibrome mou ou fibrome pendulaire) :

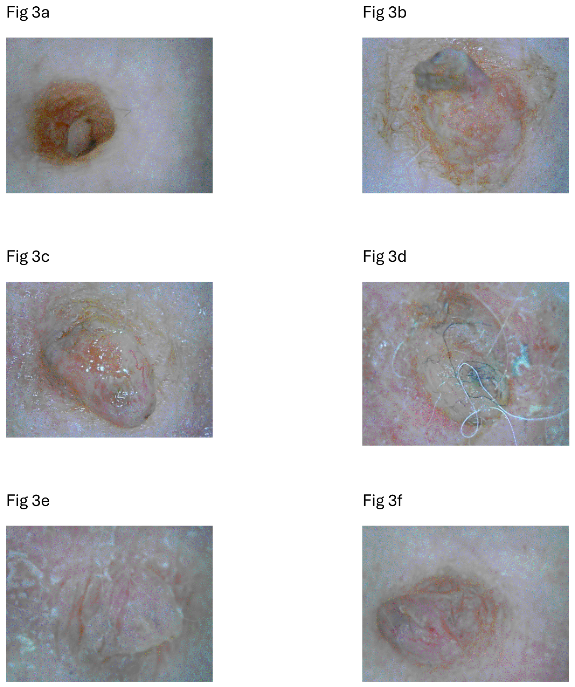

Les photographies des figures 3a-3f correspondent à ces observations :
1. J0 : avant application : souci de zoom, mais acrochordon (fibrome mous ou fibrome pendulaire) d'environ 2 mm sur pigmentation de type grain de beauté - figure 3a ;
2. J0 : après rinçage/séchage : pas de sensation/douleur spécifique, nécrose de l'extrémité de l'acrochordon (couleur noirâtre), diffusion de la pigmentation - figure 3b ;
3. J1 : Pas de sensation/douleur spécifique, la nécrose de l'extrémité de l'acrochordon (couleur noirâtre) s'était séparée ; diffusion de la pigmentation confortée ; inflammation de la microcirculation de l'acrochordon (dilatation des vaisseaux visible) - figure 3c ;
4. J3 : Pas de sensation/douleur spécifique, assèchement de l'acrochordon et disparition de la vascularisation ; diffusion de la pigmentation confirmée ; nécrose de l'épiderme autour de l'acrochordon - figure 3d ;
5. J10 : Pas de sensation/douleur spécifique, assèchement de l'acrochordon conforté et disparition de la vascularisation confortée ; disparition de la pigmentation confirmée sur l'acrochordon (léger halo de pigmentation autour du reste d'acrochordon ; nécrose de l'épiderme autour de l'acrochordon - figure 3e ;
6. J14 : Pas de sensation/douleur spécifique, assèchement de l'acrochordon conforté (très forte réduction en taille) et disparition de la vascularisation confortée ; disparition de la pigmentation confirmée sur l'acrochordon (très léger halo de pigmentation autour du reste d'acrochordon, mais très atténué par rapport à la pigmentation d'origine) - figure 3f.

### Observations sur le patient avec un Angiome rubis :

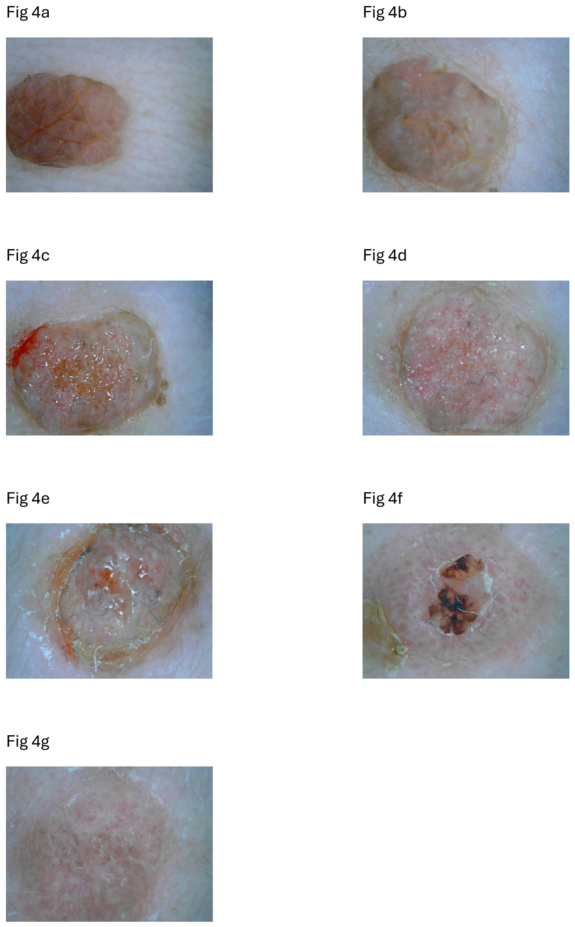

Les photographies des figures 4a-4g correspondent à ces observations :
1. J0 : avant application : Grain de beauté bombé, vascularisé et très teinté - figure 4a ;
2. J0 après rinçage/séchage : Pas de sensation/douleur spécifique, diffusion (disparition notable) de la teinte du grain de beauté ; inflammation de la vascularisation - figure 4b ;
3. J1 : Pas de sensation/douleur spécifique, diffusion (disparition notable) de la teinte du grain de beauté confortée ; inflammation de la vascularisation confortée et nécrose de la vascularisation - figure 4c ;
4. J2 : Pas de sensation/douleur spécifique, diffusion (disparition notable) de la teinte du grain de beauté confortée ; nécrose de la vascularisation confortée - figure 4d ;
5. J3 : Pas de sensation/douleur spécifique, diffusion (disparition notable) de la teinte du grain de beauté confortée ; nécrose de la vascularisation confortée, nécrose de la chair autour de la vascularisation à l'intérieur du grain de beauté-figure 4 e ;
6. J7 : Pas de sensation/douleur spécifique, diffusion (disparition notable) de la teinte du grain de beauté confortée ; nécrose de la chair autour de la vascularisation à l'intérieur du grain de beauté confirmée par une croûte -figure 4 f ;
7. J10 Pas de sensation/douleur spécifique, nécrose de l'épiderme par effet diffusion autour de la zone traitée. La teinte est quasi entièrement disparue et la peau n'est plus bombée ; l'Angiome rubis a disparu - figure 4g.

### Exemple 2.4 composition topique

Dans le tableau 4 est présentée une composition utilisable selon l'invention contenant de l'acide 6-n-pentadecylsalicylique, acide anacardique en C15 :0.

**[Tableau 4]**

| Phase | Nom commercial | % | INCI |
|---|---|---|---|
| A | Eau | Qsp | Aqua |
| C | Cutina GMS | 4,50 | Glyceryl monostearate |
| C | Alcool cétéarylique | 1,50 | Cetearyl alcohol |
| C | Amisoft HS-11P | 0,30 | Sodium stearoyl glutamate |
| B | Glycérine | 3,50 | glycerin |
| B | Gomme de xanthane FF commercialisée par Jungbunzlauer | 0,15 | xanthan gum |
| C | Huile de tournesol raffinée commercialisée par CAUVIN | 25,00 | Helianthus annuus seed oil |
| D | Acide 6-n-pentadecylsalicylique commercialisé par Sigma Aldrich Merck | 15,00 | |
| E | Acide citrique monohydraté | 0,40 | Citric acid |
| Total | | 100 | |

D'une part, les constituants de la phase A ont été mélangés à 20°C avec un mixeur mécanique à couteaux ou un rotor stator à une vitesse comprise entre 3500 et 4000 rpm durant 7 minutes pour obtenir un mélange homogénéisé. D'autre part, les constituants de la phase B ont été mélangés à 20°C avec un mixeur mécanique à couteaux ou un rotor stator à une vitesse comprise entre 3500 et 4000 rpm durant 7 minutes

Enfin la phase B a été introduite dans la phase A et les phases ont été mélangées avec un mixeur mécanique à couteaux ou un rotor stator à une vitesse de 4000 rpm durant 5-7 minutes pour obtenir une composition homogénéisée.

Le pH de la composition obtenue est ajusté à 5,0.

Le mode d'application privilégié est d'utiliser un pansement percé qui délimite la zone à traiter, « l'œillet » ou le « trou » du pansement indique la zone à traiter, d'introduire la composition dans le trou ou l'œillet au contact de la peau et recouvrir le trou ou l'œillet avec un sparadrap, laisser le dispositif en place durant au minimum 30 minutes et jusque 24 h, puis éliminer le dispositif appliqué et éventuellement, rincer la peau à l'eau claire de l'excédent de produit.

Cette composition a été appliquée dans les mêmes conditions que celles de la composition de l'exemple 2.3 ci-dessus, les effets observés suite aux applications sont similaires à ceux de la composition de l'exemple 2.3. Il a néanmoins été noté que la composition de l'exemple 2.4 présente des aptitudes supérieures pour le curetage profond, sur des plaies plus profondes, avec une optimisation de l'effet anti-douleur.

### Exemple 2.5 composition sous forme de dispositif transdermique (patch)

Dans le tableau 5 est présentée une composition de « patch » utilisable selon l'invention.

**[Tableau 5]**

| Phase | Nom commercial | % | INCI |
|---|---|---|---|
| A | Myritol 318 commercialisé par BASF | 70,00 | Caprylic :capric triglyceride |
| A | Vitamine C TetraE commercialisée par Ephyla | 5,0 | Ascorbyl tetraisopalmitate |
| B | Extrait pur (98%) de coques de cajou obtenu à l'exemple 1.2 | 25,00 | *Anarcardium occidentale L* (Cashew shell) extract |
| Total | | 100 | |

Les constituants des phases A et B ont été mélangés à 20°C sous agitation lente avec une simple hélice à une vitesse de 300-800 rpm.

Cette composition est destinée à être appliquée en patch sur une durée de 2 à 6h. Cette composition vise les couches profondes du derme et l'hypoderme, elle vise à faire coaguler les cellules pour une mise en nécrose. Elle a été appliquée pendant 2 à 6 heures selon les personnes (volontaires), puis rincée à l'eau claire. Après 48 à 96h, les adipocytes, et plus particulièrement les kystes graisseux ou encore les tissus contaminés par une verrue disparaissent par phénomène de nécrose.

Cette composition est utilisable dans des traitements visant à éliminer, par nécrose, la graisse hypodermique, plus particulièrement les kystes graisseux ou encore pour éliminer, par nécrose, les verrues.

Sur d'autres volontaires présentant des imperfections cutanées de type petits angiomes ou taches rubis plates ou surélevées ou les vaisseaux sanguins apparents disgracieux, cette composition a été appliquée pendant 2 à 3 heures selon les personnes (volontaires), puis rincée à l'eau claire. Après 48 à 96h, les défauts mentionnés disparaissent par phénomène de nécrose.

### Exemple 2.6 composition sous forme de dispositif transdermique

Dans le tableau 6 est présentée une composition de « patch » utilisable selon l'invention.

**[Tableau 6]**

| Phase | Nom commercial | % | INCI |
|---|---|---|---|
| A | Myritol 318 commercialisé par BASF | 70,00 | Caprylic/ capric triglyceride |
| A | Vitamine C TetraE commercialisée par Ephyla | 5,0 | Ascorbyl tetraisopalmitate |
| B | Extrait concentré (73%) de coques de cajou obtenu à l'exemple 1.1 | 25,00 | *Anarcardium occidentale L* (Cashew shell) extract |
| Total | | 100 | |

Les constituants des phases A et B ont été mélangés à 20°C sous agitation lente avec une simple hélice à une vitesse de 300-800 rpm.

Cette composition permet de traiter les couches profondes du derme et l'hypoderme, elle permet de faire coaguler les cellules pour une mise en nécrose, elle a été appliquée pendant 2 à 6 heures selon les personnes (volontaires), puis rincée à l'eau claire. Après 48 à 96 heures, les adipocytes, et plus particulièrement les kystes graisseux ou encore les tissus contaminés par une verrue disparaissent par phénomène de nécrose.

Cette composition est utilisable dans les traitements visant à éliminer par nécrose la graisse hypodermique, plus particulièrement les kystes graisseux et/ou dans les traitements visant à éliminer les verrues par nécrose.

Sur d'autres volontaires présentant des imperfections cutanées de type petits angiomes ou taches rubis plates ou surélevées ou les vaisseaux sanguins apparents disgracieux, cette composition a été appliquée pendant 2 à 3 heures selon les personnes (volontaires), puis rincée à l'eau claire. Après 48 à 96h, les défauts mentionnés disparaissent par nécrose.

### Exemple 2.7 composition injectable

Dans le tableau 7 est présentée une composition injectable utilisable selon l'invention

**[Tableau 7]**

| Phase | Nom commercial | % | INCI |
|---|---|---|---|
| A | EPHYSTER ECR | 60,00 | Ethyl linoleate |
| | commercialisé par Ephyla | | |
| A | TecFis QF commercialisé par Fismerlecithin | 5,0 | Lecithin |
| B | Extrait concentré (73%) de coques de cajou obtenu à l'exemple 1.1 | 35,00 | *Anarcardium occidentale L* (Cashew shell) extract |
| Total | | 100 | |

Les constituants des phases A et B ont été mélangés à 20°C sous agitation lente avec une simple hélice à une vitesse de 300-800 rpm. Puis le mélange a été soumis à une filtration stérilisante.

Cette composition permet de traiter les couches profondes du derme et de l'hypoderme.

Chez les volontaires ayant des verrues profondes dans le tissu cutané, notamment au niveau de la plante des pieds, il est observé une nécrose de la verrue et du tissu contaminé environ 96h après l'injection localisée.

Chez les volontaires ayant une hémorragie sous cutanée, l'injection de la composition sur la zone permet de stopper, en quelques minutes, l'hémorragie (arrêt du grossissement lié au saignement interne) par coagulation des cellules endothéliales lésées ; il est par ailleurs observé que l'hématome est moins intense et disparaît rapidement.

Chez les volontaires ayant une douleur localisée, l'injection avec aiguille directement sur la zone à traiter permet de réduire sensiblement la douleur après 30 minutes et complètement après 2 heures, et ceci de manière durable.

### Exemple 2.8 composition injectable

Dans le tableau 8 est présentée une composition injectable utilisable selon l'invention

**[Tableau 8]**

| Phase | Nom commercial | % | INCI |
|---|---|---|---|
| A | EPHYSTER ECR commercialisé par Ephyla | 60,00 | Ethyl linoleate |
| A | TecFis QF commercialisé par Fismerlecithin | 5,0 | Lecithin |
| B | Extrait pur (98%) de coques de cajou obtenu à l'exemple 1.2 | 35,00 | *Anarcardium occidentale L* (Cashew shell) extract |
| Total | | 100 | |

Les constituants des phases A et B ont été mélangés à 20°C sous agitation lente avec une simple hélice à une vitesse de 300-800 rpm. Puis le mélange a été soumis à une filtration stérilisante.

Cette composition permet de traiter les couches profondes du derme et de l'hypoderme.

Chez les volontaires ayant des verrues profondes dans le tissu cutané, notamment au niveau de la plante des pieds, il est observé une nécrose de la verrue et du tissu contaminé environ 96h après l'injection localisée.

Chez les volontaires ayant une hémorragie sous cutanée, l'injection de la composition sur la zone permet de stopper, en quelques minutes, l'hémorragie (arrêt du grossissement lié au saignement interne) par coagulation des cellules endothéliales lésées ; il est par ailleurs observé que l'hématome est moins intense et disparaît rapidement.

Chez les volontaires ayant une douleur localisée, l'injection avec aiguille directement sur la zone à traiter permet de réduire nettement la douleur après 30 minutes et complètement après 2 heures, et ceci de manière durable.

### Exemple 2.9 composition injectable

Dans le tableau 9 est présentée une composition injectable utilisable selon l'invention

**[Tableau 9]**

| Phase | Nom commercial | % | INCI |
|---|---|---|---|
| A | EPHYSTER ECR commercialisé par Ephyla | 60,00 | Ethyl linoleate |
| A | TecFis QF commercialisé par Fismerlecithin | 5,0 | Lecithin |
| B | Acide 6-n-pentadecylsalicylique commercialisé par Sigma Aldrich Merck | 35,00 | |
| Total | | 100 | |

Les constituants des phases A et B ont été mélangés à 20°C sous agitation lente avec une simple hélice à une vitesse de 300-800 rpm. Puis le mélange a été soumis à une filtration stérilisante.

Cette composition permet de traiter les couches profondes du derme et de l'hypoderme. Chez les volontaires ayant des verrues profondes dans le tissu cutané, notamment au niveau de la plante des pieds, il est observé une nécrose de la verrue et du tissu contaminé environ 96 heures après l'injection localisée.

Chez les volontaires ayant une hémorragie sous cutanée, l'injection de la composition sur la zone permet de stopper, en quelques minutes, l'hémorragie (arrêt du grossissement lié au saignement interne) par coagulation des cellules endothéliales lésées ; il est par ailleurs observé que l'hématome est moins intense et disparaît rapidement.

Chez les volontaires ayant une douleur localisée, l'injection avec aiguille directement sur la zone à traiter permet de réduire sensiblement la douleur après 30 minutes et complètement après 2 heures, et ceci de manière durable.

### Exemple 3.1-Etude sur explants de peau humaine

### Principe de l'étude

Pour cette étude un explant de peau humaine congelé a été découpé en disques de 10⁻² m (1 cm) de diamètre.

5 séries échantillons d'explants à tester -en triplicat- ont été préparées :
- série 1 : échantillons (disques) témoins,
- série 2 : échantillons (disques) traités pendant 30 minutes ;
- série 3 : échantillons (disques) traités pendant 60 minutes ;
- série 4 : échantillons (disques) traités pendant 120 minutes ;
- série 5 : échantillons (disques) traités pendant 240 minutes.

Le traitement des disques des séries 2-5 a consisté à appliquer 500 mg de la formule du tableau 10 ci-dessous sur chaque explant.

**[Tableau 10]**

| Phase | Nom commercial | % | INCI |
|---|---|---|---|
| B | Eau | Qsp | Aqua |
| A | Frametime CXG commercialisé par Ephyla | 4,5 | Bentonite & xanthan gum & Sodium stearoyl glutamate & citric acid |
| A | OLO3D commercialisé par Ephyla | 4,0 | Stearalkonium Bentonite |
| B | Extrait concentré (73%) de coques de cajou obtenu à l'exemple 1.1 | 30,0 | *Anarcardium occidentale L* (Cashew shell) extract |
| Total | | 100 | |

Cette formule a été préparée de la façon suivante.

D'une part, les constituants de la phase A ont été mélangés à 20°C avec un mixeur mécanique à couteaux ou un rotor stator à une vitesse comprise entre 3500 et 4000 rpm durant 7 minutes pour obtenir un mélange homogénéisé. D'autre part, les constituants de la phase B ont été mélangés à 20°C avec un mixeur mécanique à couteaux ou un rotor stator à une vitesse comprise entre 3500 et 4000 rpm durant 7 minutes

Enfin la phase B a été introduite dans la phase A et les phases ont été mélangées avec un mixeur mécanique à couteaux ou un rotor stator à une vitesse de 4000 rpm durant 5-7 minutes pour obtenir une composition homogénéisée.

Le traitement des disques des séries 2-5 a été effectué de la manière suivante : 500 mg de formule du tableau 10 ci-dessus ont été appliqués sur chaque échantillon, un échantillon correspondant à un disque d'explant placé en condition de survie sur milieu physiologique (plaque de culture 6 puits).

Après traitement pendant le délai indiqué ci-dessus (entre 30 minutes pour la série 2 et 240 minutes pour la série 5), les échantillons ont été retirés pour être rincés avec de l'eau physiologique (de sorte à retirer le traitement à la surface de la peau). Les échantillons ont ensuite été remis en condition de survie sur milieu physiologique (dans étuve CO₂) ; puis congelés.

L'examen histologique des disques de peau humaine a été réalisé par le laboratoire d'immunohistologie après mise en coloration HES (Hémalun-Eosine-Safran). L'ensemble des structures épidermiques et dermiques a été évalué.

### Conclusions:

L'analyse histologique a mis en évidence une évolution progressive des altérations cutanées avec le temps.

Aucun changement majeur n'a été noté sur les disques traités pendant 30 min, mais l'apparition d'une possible élastose dans le derme superficiel a été notée sur les disques traités pendant 60 min.

À 120 min, des altérations nucléaires sont notées dans l'épiderme sans inflammation significative.

À 240 min, une nécrose épidermique associée à une infiltration inflammatoire plus marquée est observée.

Ainsi, la formule testée montre une action sur le derme et l'épiderme, l'action spécifique sur l'épiderme à partir de 120 minutes qui se confirme à 240 minutes par une nécrose ou abrasion de l'épiderme

### Exemple 3.2-Effet de l'extrait pur

3 patients ont été traités avec l'extrait pur préparé à l'exemple 1.2 ci-dessus : 1 patient avec un acrochordon (fibrome mou ou fibrome pendulaire) au niveau du cou, 1 patient avec un Angiome rubis vascularisé sur la joue, 1 patient avec Naevus dysplasique sur omoplate.

### Exemple 3.2 b-traitements

### Matériel et méthode :

Les observations ont été effectuées avec un appareil dermatoscope de type FIREFLY Digital DE300 (zoom x60).

### Préparation des patients

La préparation des 3 patients à traiter a été effectuée de la même façon qu'à l'Exemple 2.1 b-traitements.

### Application du traitement

L'application du traitement a été effectuée de la même façon qu'à l'Exemple 2.1 b-traitements.

### Soin du traitement

Le soin du traitement a été effectuée de la même façon qu'à l'Exemple 2.1 b-traitements.

### Suivi et documentation

1. Observation quotidienne avec le dermatoscope de type FIREFLY Digital DE300 (zoom x60) pendant les 4 premiers jours.
2. Puis observation tous les 2-3 jours jusqu'au jour J+14.

### Observations sur un patient avec acrochordon (fibrome mou ou fibrome pendulaire) au niveau du cou

J0 : après 4h ; la zone traitée était brunie par un effet de cornéification ; l'acrochordon s'était rigidifié et décroché ; pas de sensation particulière ;
J1 : Pas de sensation particulière ; la zone traitée s'était cornéifiée ;
J2 : croûte en formation sur la zone traitée ; le pourtour est rouge ; légère démangeaison ;
J3 : idem à J2 avec effet hyperémiant de diffusion autour de la zone traitée ; léger gonflement sur le pourtour ; légère démangeaison ;
J5 : la zone traitée avait évolué en croûte dure et profonde ; le pourtour était toujours rouge ; absence de démangeaison ;
J7 : la zone traitée était devenue une croûte déjà sèche ; absence de démangeaison ;
J10 : la croûte se décollait sur 30-35% de sa surface, notamment sur son pourtour ;
J14 : la croûte s'était décollée ; la peau était saine sans cicatrice apparente ; la pigmentation et l'acrochordon (fibrome mou ou fibrome pendulaire) avaient disparu ;

### Observations sur le patient avec un Angiome rubis vascularisé sur la joue

J0 : après 4h ; la zone traitée était brunie par un effet de cornéification ; la vascularisation semblait enflammée avec saignement en coagulation ; petite sensation de picotement ;
J1 : la zone traitée s'était cornéifiée ; la vascularisation avait coagulé et une croûte était en formation ; pas de sensation particulière ;
J2 : idem à J1 avec confirmation d'une croûte en formation ;
J3 : idem à J2 avec léger effet hyperémiant de diffusion autour de la zone traitée et de la croûte formée ; petite démangeaison ;
J5 : la zone traitée avait évolué en croûte ; le pourtour était rouge ; petite démangeaison ;
J7 : la zone traitée était devenue une croûte qui séchait ; pas de sensation particulière ;
J10 : la croûte était sèche et elle se décollait significativement sur son pourtour ;
J14 : la croûte s'était décollée ; la peau était saine sans cicatrice apparente ; la pigmentation et l'Angiome rubis avaient disparu.

### Observations sur le patient avec Naevus dysplasique sur omoplate

J0 : après 4 h ; la pigmentation du Naevus dysplasique était réduite en un amas ; la vascularisation semblait enflammée sur toute la zone du Naevus ; sensation de picotement ;
J1 : la zone traitée s'était cornéifiée, les traces de la pigmentation du Naevus avaient complétement disparu ; la vascularisation avait coagulé et s'était rigidifiée ; pas de sensation particulière ;
J2 : idem à J1 ; léger effet hyperémiant de diffusion autour de la zone traitée ; légère sensation de démangeaison ;
J3 : idem à J2 avec effet hyperémiant de diffusion autour de la zone traitée ; cette zone était sensiblement gonflée ; légère sensation de démangeaison ;
J5 : la zone traitée avait évolué en croûte ; le pourtour était en hyperémie rouge ; sensation de démangeaison ;
J7 : la zone traitée était devenue une croûte qui séchait ; le pourtour était en hyperémie rouge comme en J5 ; très légère sensation de démangeaison ;
J10 : la croûte était sèche et semblait se décoller sur son pourtour ; le pourtour présentait toujours une hyperémie ; pas de sensation particulière ;
J14 : la croûte s'était décollée ; la peau était saine sans cicatrice apparente ; le Naevus dysplasique avait totalement disparu ; une hyperémie sur le pourtour était toujours présente ; pas de sensation particulière.

Il s'agit donc d'une coagulation entrainant une exfoliation ; le traitement est particulièrement efficace pour éliminer par nécrose et exfoliation les acrochordons (fibromes mous ou fibromes pendulaires), les cicatrices, les vergetures, les lésions pigmentaires (mélasma), les Naevus notamment dysplasiques, les taches de vieillesse ou lentigos, les grains de beauté disgracieux tels que les angiomes rubis. La coagulation des micro-vaisseaux sanguins observée dans le cas des angiomes rubis permet aussi des applications visant à stopper les saignements notamment lors de coupures cutanées touchant le derme et/ou l'hypoderme.

Il est à préciser que le contenu de cette composition pénètre à travers l'épiderme pour rejoindre le derme également, ce qui permet, chez d'autres volontaires, au niveau de la jonction dermo-épidermique, d'inhiber la douleur par nécrose cellulaire des cellules sous inflammation au niveau des terminaisons nerveuses, cette composition est donc particulièrement indiquée pour le curetage des plaies, en ce cas l'application n'excède pas 3 h et au minimum 1h : l'application en cataplasme agit, sans action mécanique, comme un curetage.

L'extrait pur préparé à l'exemple 1.2 ci-dessus a été étudié par immunofluorescence avec marquage P53 et Caspase-3

### Immunofluorescence avec marquage P53

L'analyse quantitative du marqueur de P53 qui est la protéine de différenciation et réparation épidermique a été effectuée selon le protocole ci-dessous.

Dans le tableau 11 est présenté l'expression différentielle de P53 après traitement par l'extrait pur.

**[Tableau 11]**

| Compartiment | Témoin | Traité 240 min | Variation (%) |
|---|---|---|---|
| Épiderme | 58,00 | 62,33 | +7.46% |
| Derme | 55,76 | 55,82 | +0.11 % |

L'augmentation significative de P53 dans l'épiderme (+7.46%) *vs* la stabilité quasi-totale dans le derme (+0.11 %) démontre que l'extrait éthanolique pur exerce une action qui est sélective et différenciante sur les kératinocytes épidermiques. L'augmentation significative de P53 dans l'épiderme confirme que l'extrait pur induit spécifiquement un processus de turnover épidermique contrôlé, caractéristique d'une exfoliation différenciée.

### Immunofluorescence avec marquage Caspase-3

L'analyse quantitative du marqueur Caspase-3 qui est un marqueur d'apoptose a été effectuée selon le protocole ci-dessous.

### Protocole

Dans le tableau 12 est présenté l'expression de la Caspase-3 après traitement par l'extrait pur.

**[Tableau 12]**

| Compartiment | Témoin | Traité 240 min | Variation (%) |
|---|---|---|---|
| Épiderme | 51.59 | 52.55 | +1.86% |
| Derme | 52.58 | 54.63 | +3.90% |

### Résultats

L'augmentation modérée de Caspase-3 dans l'épiderme (+1.86%) confirme l'engagement des kératinocytes dans un processus apoptotique contrôlé, cohérent avec le mécanisme d'exfoliation observé histologiquement.

## Revendications

1. Composition pharmaceutique comprenant de l'acide anacardique pour son utilisation en tant que coagulant cellulaire.

2. Composition pour son utilisation selon la revendication 1 dans laquelle l'acide anacardique est choisi parmi l'acide 6-n-pentadecylsalicylique, l'acide 6-[8(Z)-n-pentadecenyl] salicylique, l'acide 6- [8(Z), 11(Z)-n-pentadecadienyl] salicylique, l'acide 6- [8(Z), 11(Z), 14(Z)-n-pentadecatrienyl] salicylique ainsi que leurs mélanges en toutes proportions.

3. Composition pour son utilisation selon la revendication 1 ou 2 dans laquelle l'acide anacardique est choisi parmi l'acide 6-n-pentadecylsalicylique, l'acide 6-[8(Z)-n-pentadecenyl] salicylique, ainsi que leurs mélanges en toutes proportions.

4. Composition pour son utilisation selon la revendication 1 à 3 comprenant un extrait de coques de noix de cajou ou *Anacardium occidentale L.,* ledit extrait contenant de l'acide anacardique.

5. Composition pour son utilisation selon l'une quelconque des revendications précédentes comprenant de 0,1% à 100,0 % en poids d'acide anacardique par rapport au poids total de la composition.

6. Composition pour son utilisation selon l'une quelconque des revendications précédentes contenant de 0,1 à 99,9% en poids, par rapport au poids total de la composition, d'une phase huileuse comprenant au moins une huile choisie parmi les huiles végétales, le squalane, le squalène, les esters de corps gras notamment végétaux.

7. Composition pour son utilisation selon la revendication précédente dans laquelle la phase huileuse végétale comprend au moins une huile végétale choisie dans le groupe formé par l'huile de tournesol, l'huile de colza, l'huile d'olive, l'huile de cameline, l'huile d'arachide, l'huile de coco, l'huile de pépin de raisin, l'huile de ricin, l'huile d'argan, l'huile de Djansang, l'huile de dattier du désert, l'huile de chanvre, l'huile de nigelle, l'huile de figue de barbarie, l'huile de macadamia, l'huile de soja, l'huile de palme et de palmier, l'huile de Tamanu, l'huile de Kukui, l'huile de sésame, l'huile de lin, l'huile de noix, l'huile de noisette, l'huile d'amande, l'huile de baobab, l'huile de fruit de la passion, l'huile de noix du Brésil, l'huile d'hibiscus, l'huile de pépin de courge, l'huile de Luffa, l'huile de Carapa, l'huile d'Onagre, l'huile de bourrache, huile d'avocat, l'huile d'argousier, l'huile de noyau d'abricot, l'huile de noyau de cerise, l'huile de pépin de pomme, l'huile de grenade, l'huile de Jojoba, l'huile de rose musquée, l'huile de prune, le beurre de karité, le beurre de cacao, le beurre de Kokum, le beurre de mangue, le beurre de moabi, le beurre de Karanja, le beurre de Tucuma, le beurre de Cupuaçu, le beurre de Buriti, le beurre de Murumuru, le beurre de Kombo, le beurre de Kpangnan et les triglycérides notamment d'acides caprylique/caprique.

8. Composition pour son utilisation selon l'une quelconque des revendications précédentes dans laquelle ladite composition comprend une émulsion de Pickering H/E contenant une phase aqueuse, une phase huileuse végétale et un phyllosilicate organomodifié.

9. Composition pour son utilisation selon la revendication précédente dans laquelle le phyllosilicate organomodifié est présent en une quantité allant de 2% à 20%, de préférence 2,5% à 10% et de manière préférée 3,5% à 8% en poids par rapport au poids total de la composition.

10. Composition pour son utilisation selon l'une quelconque des revendications précédentes comprenant de 0,001 à 20 % en poids d'au moins un agent choisi parmi les agents humectants, les épaississants, les agents de texture, les émulsifiants, les agents dispersants, les agents moussants, les émollients, les conservateurs, les colorants, les extraits de plantes, les fibres végétales, les minéraux, les agents correcteurs de pH, les principes actifs et les parfums.

11. Composition pour son utilisation selon l'une quelconque des revendications précédentes pour son utilisation pour traiter les adipocytes et conférer un effet anti-cellulite à la peau, et/ou permettre la réduction de lipomes.

12. Composition pour son utilisation selon l'une quelconque des revendications 1 à 11 pour son utilisation pour traiter l'environnement cellulaire en inflammation autour des cellules nerveuses et agir en tant qu'agent anti-douleur.

13. Composition pour son utilisation selon l'une quelconque des revendications 1 à 11 pour son utilisation pour réduire une fibrose ou un kyste.

14. Composition pour son utilisation selon l'une quelconque des revendications 1 à 11 pour son utilisation pour exfolier la peau et/ou les défauts de surfaces notamment les lentigos, acrochordons, les kystes cutanés, les molluscums, les kératoses et les xanthélasmas.

15. Composition pour son utilisation selon la revendication précédente pour son utilisation pour exfolier la peau dans au moins un traitement choisi dans le groupe formé par :
le traitement des cicatrices en creux, le curetage et le traitement des verrues, le traitement des boutons d'herpès, le traitement des acrochordons, le traitement des taches pigmentaires ou lentigos, le traitement des kératoses, le traitement des xanthélasmas, le curetage des plaies, le traitement des télangiectasies, le traitement des varicosités, le traitement des angiomes, le traitement des taches rubis, le traitement des vaisseaux sanguins superficiels de la peau.
